# DEMANDE DE BREVET EUROPEEN

(11) **EP 2 662 110 A1**
(43) Date de publication de la demande: **13.11.2013**
(21) Numéro de dépôt: 12167545.8
(22) Date de dépôt: 10.05.2012
(51) Int. Cl.: A61M 37/00, A61M 5/20, A61M 5/32

(54) **Dispositif et méthode pour l'insertion d'aiguilles**

(71) Demandeur: DEBIOTECH S.A., 1004 Lausanne (CH)
(72) Inventeur: Da Ros, Jérôme, 74200 Thonon les Bains (FR); Neftel, Frédéric, 1005 Lausanne (CH)
(74) Mandataire: Grosfillier, Philippe

(57) **Abrégé**

Dispositif pour l'insertion d'aiguilles comprenant un boitier (1), un piston (2), au moins une aiguille creuse (10), des moyens de propulsion comprenant des moyens de retenue (3a) pour rendre temporairement solidaires les moyens de propulsion (3, 4) au piston (2), le dispositif étant configuré de manière à ce que le piston (2) atteigne une première position, formant une distance D1 entre la face distale du piston (2) et l'extrémité proximale du boitier (1) ; une deuxième position, formant une distance D2; puis une troisième position, formant une distance D3 ; D3 étant inférieure ou égale à D1 et à D2. Le dispositif comportant en outre des moyens de pression destinés à autoriser au moins un recul maîtrisé du piston (2) lors du passage de la première à la deuxième position puis à la troisième position.

## Description

### Domaine de l'invention

La présente invention concerne l'insertion d'aiguilles, en particulier de micro-aiguilles. Celle-ci peut être utilisée pour l'injection intradermique ou sous-cutanée de solutions.

### Etat de la technique

Des dispositifs pour l'insertion de micro-aiguilles sont divulgués dans les documents suivants : US 6 743 211, US 4 886 499, US 7 083 592 et US 20100030148.

### Lien avec demande antérieure

Cette demande intègre tous les éléments de description de la demande PCT/IB2011/055256 et de la demande EP 10193557.5 et apporte des améliorations substantielles du dispositif précédemment décrit dans lesdites demandes antérieures.

### Description générale de l'invention

La présente invention constitue une amélioration par rapport aux méthodes et dispositifs de l'état de la technique.

Elle concerne un dispositif et une méthode tels que définis dans les revendications.

L'invention permet notamment de percuter un tissu par une ou plusieurs micro-aiguilles à des vitesses relativement élevées, typiquement de l'ordre de 3 à 15 m/s (typiquement 7m/s), tout en laissant le tissu amortir le choc sur une certaine distance, ce qui a pour effet d'améliorer la perforation du tissu et de ramener cette dernière à un état stable d'équilibre, minimisant de la sorte les contraintes exercées sur le tissu du patient.

Dans le cadre de la présente invention, une fois insérées, les micro-aiguilles sont maintenues contre le tissu grâce à une légère pression afin que ces dernières ne puissent être éjectées en arrière du fait de la réaction élastique du tissu après l'impact des micro-aiguilles. Cette légère pression a également pour but de maintenir les micro-aiguilles afin d'éviter toute fuite durant l'injection. Toutefois, cette légère pression ne doit pouvoir s'opposer à la formation de papules induites par l'injection intradermique, ie la légère pression exercée doit permettre un léger retrait ou déplacement des aiguilles avec le tissu au moment de la formation de cette papule.

La présente invention permet en particulier de réduire la pression exercée sur la ou les aiguilles ou, de façon plus générale, sur le piston ; ce dernier ayant pour fonction de supporter les aiguilles. Cette réduction de pression est rendue possible grâce à des moyens de pression maintenant les aiguilles sur le tissu une fois que celles-ci y ont pénétré. Ces moyens de pression ne sont toutefois pas trop importants afin d'autoriser la création de papule durant l'injection.

Le dispositif selon l'invention offre aussi l'avantage de pouvoir être utilisé dans n'importe quelle orientation par rapport à la peau.

Dans le cadre de la présente invention, l'expression «extrémité distale» désigne l'extrémité qui est la plus éloignée de la main de l'opérateur et l'expression «extrémité proximale» désigne l'extrémité qui est la plus rapprochée de la main de l'opérateur.

Le dispositif selon l'invention permet également, selon les caractéristiques du tissu et/ou la manipulation par l'opérateur, d'avoir D1 supérieure à D2 ou inversement D1 inférieure ou égale à D2

Dans une réalisation possible, le dispositif a un corps de forme allongée dans lequel, un piston est monté coulissant et restreint à un mouvement dans l'axe de l'orientation principale, l'aiguille faisant saillie sur ledit piston. Dans une réalisation possible, les moyens de propulsion sont constitués d'un propulseur logeant un ressort, (ou un élastique ou une lame élastique) et étant temporairement solidaire du piston grâce à des moyens de retenue.

Dans un mode de réalisation préféré, le propulseur dispose de clips adaptés pour retenir le piston. Lesdits clips sont configurés pour accompagner le piston en direction du tissu - grâce au propulseur lors du déclenchement de l'insertion - et pour se déclipser dès que le piston - lors de l'insertion dans le tissu - rencontre une force de sens inverse et d'intensité prédéterminée.

Lors du déclenchement du dispositif, le propulseur entraine le piston en direction du tissu créant une force F1. Dès que l'aiguille entre en contact avec le tissu, le tissu va générer une contre force (nommée F2) - dans le sens opposé de l'insertion - sur l'ensemble aiguille-piston. F2 continue à croitre tant que le piston continue sa course (dans le sens F1), jusqu'à atteindre une valeur prédéterminée dont l'intensité est telle qu'une force radiale en résulte et qui a pour effet de libérer le piston des moyens de propulsion.

Les moyens de retenue peuvent être façonnés en fonction des caractéristiques des moyens de propulsion et des caractéristiques du tissu dans lequel les aiguilles doivent être insérées.

Dans un autre mode de réalisation, les moyens de retenue se désolidarisent à cause de l'impact ou par d'autres moyens tels qu'une butée ou de façon manuelle afin que le propulseur n'exerce plus de pression sur l'ensemble aiguille-piston avant le début de l'injection de la solution.

Lors de l'impact des aiguilles contre le tissu, l'ensemble aiguilles-piston peut être projeté dans le sens opposé de l'insertion et causer ainsi le délogement total ou partiel des aiguilles bien que ces dernières aient été initialement correctement insérées. De plus, durant l'injection, selon la direction du ou des canaux et la forme des aiguilles, les aiguilles peuvent sortir totalement ou partiellement de leurs logements. Afin d'assurer la bonne insertion des aiguilles et de limiter les fuites de la solution à injecter, des moyens de pression permettent aux aiguilles de rester insérées dans le tissu après l'insertion et pendant l'injection.

Ainsi, dans le cadre de la présente invention, dès que l'aiguille entre en contact avec le tissu, une pression de l'ensemble aiguille-piston contre le tissu est exercée par des moyens de pression. Toutefois, pour une injection optimale de la solution dans ou à travers le tissu, ces moyens de pression doivent avoir une force suffisamment faible du début à la fin de la formation de la papule afin de ne pas limiter la formation de cette papule au cours de l'injection.

Exprimé différemment, lesdits moyens de pression exercent une force selon une direction correspondant à l'orientation principale dans le sens de l'insertion, c'est-à-dire en direction du tissu, sans pour autant gêner la formation de la papule.

Cette force peut être générée par un seul élément ou plusieurs éléments qui peuvent être une tubulure, un ressort, un élastique, une cartouche de gaz, de l'air comprimé, une force électromagnétique, la génération de gaz par réaction chimique entre au moins deux composés, une lame élastique, le poids de l'ensemble piston-aiguille et/ou différents frottements.

Dans un autre mode de réalisation, les moyens de pression exercent une force dès l'activation des moyens de propulsion. Le ressort des moyens de propulsion peut se coupler aux moyens de pression pour former la source d'énergie nécessaire pour les moyens de propulsion.

Dans un autre mode de réalisation, les moyens de propulsion sont eux-même responsables de la force résiduelle en fin de course. Cela peut, par exemple, correspondre à la fin de course d'un ressort ou d'un élastique utilisé pour la propulsion du piston, le dit ressort ou élastique étant calculé de telle façon que cette force résiduelle en fin de course corresponde à la force résiduelle nécessaire.

Dans un autre mode de réalisation, les moyens de pression peuvent également servir à propulser le liquide dès que les micro-aiguilles sont logées dans le tissu du patient et que les moyens de rétention sont libérés, réalisant ainsi un auto-injecteur.

De préférence, les moyens de propulsion exercent une force dans le sens correspondant à l'insertion et d'une valeur supérieure ou égale aux moyens de pression.

Dans un mode de réalisation préféré, le dispositif est façonné de manière à ce que tout appui du dispositif contre le tissu n'exerce pas - ou de façon marginale - de pression sur l'ensemble aiguille-piston de sorte que la formation de la papule ne puisse être gênée. L'extrémité distale du dispositif est composée d'une zone de contact en forme de bague ou de pieds stabilisateurs permettant de positionner le dispositif sur le tissu (14) selon un angle désiré et de façon suffisament éloigné n'influant pas, ou peu, sur les caractéristiques mécaniques du tissu (14).

Dans une réalisation possible, les aiguilles du dispositif sont des micro-aiguilles. Par micro-aiguilles, on entend des aiguilles dont les dimensions sont adaptées pour cibler de préférence le territoire intradermique. Cette zone a une épaisseur variable selon le patient ainsi que selon l'emplacement sur le corps d'un même patient. Elle est de l'ordre de quelques centaines de microns à quelques millimètres. La micro-aiguille pourra cependant être un peu plus longue que l'épaisseur maximum de cette zone pour tenir compte de la pénétration des micro aiguilles dans le tissu qui pourrait n'être que partielle.

De préférence, le dispositif dispose d'une butée limitant la course du piston. Cette butée est positionnée de sorte que le piston peut sortir en sailli de l'extrémité distale du dispositif. Cette butée peut également permettre de désolidariser le piston du propulseur.

De préférence, le dispositif dispose d'une butée limitant la course du propulseur, de sorte que le propulseur ne puisse exercer de pression sur le piston durant l'injection.

Dans le cadre de cette invention, l'injection intradermique peut induire la formation d'une papule qui correspond à la déformation du tissu suite au stockage de la solution injectée dans le tissu, notamment lors d'une injection type bolus. A relever que la création et le maintien d'une papule sont souhaitables pour assurer une injection optimale dans le tissus ciblé. C'est pourquoi la présente invention permet à une telle papule de se former et de rester en place le temps nécessaire à la bonne diffusion de la substance injectée.

Dans un mode de réalisation, l'injection peut être déclenchée automatiquement ou manuellement une fois la ou les aiguilles insérées dans le tissu, mais préférentiellement elle commencera une fois que le piston soit revenu en deuxième position, c'est-à-dire lorsque le propulseur n'exerce plus de force contre le tissu.

Dans un mode de réalisation préféré, la seringue est en connection fluidique avec les aiguilles.

L'invention concerne également une méthode d'insertion et d'injection grâce à une ou des aiguilles, notamment de micro-aiguilles et notamment dans le derme, où l'aiguille - dotée d'un mouvement de translation selon une direction correspondant à l'orientation principale - peut décélérer soudainement ou progressivement dans le tissu du fait de l'élasticité de celui-ci sur une certaine longueur tout en assurant in fine un maintien de l'aiguille après l'insertion et durant l'injection grâce à une très légère pression de l'aguille contre le tissu. L'ensemble aiguille-piston peut exercer une pression contre le tissu durant l'insertion de telle sorte que l'ensemble aiguille-piston peut s'enfoncer dans le tissu où l'aiguille pénètre le tissu et la face distale du piston pousse le tissu. La profondeur de l'insertion de l'aguille est assurée par ladite face distale du piston limitant la profondeur d'insertion lorsque le piston est en contact avec le tissu.

Suite à ce mouvement d'insertion, la force exercée sur l'ensemble aiguille-piston est réduite, permettant d'exercer plus qu'une force minime (exercée par des moyens de pression) nécessaire pour maintenir l'aiguille insérée à une profondeur optimale pour l'injection de la solution. Ladite force minime doit être inférieure à la force qui sera générée par la formation de la papule et de sens opposée permettant ainsi un recul de l'ensemble aiguille-piston induit par la formation de la papule durant l'injection. Ainsi, lors de l'injection, l'aiguille reste solidaire de la papule en se déplaçant avec elle lors de sa croissance, tout en opposant ladite force minime.

Dans le cadre de cette invention, ladite force minime doit empêcher que le rétablissement à son équilibre du tissu ne fasse ressortir l'aiguille par inertie, et que ladite force minime n'empêche l'injection. En particulier, ladite force minime ne doit pas créer de pression à l'intérieur du tissu qui empêcherait ou limiterait la bonne diffusion de la substance devant être infusée.

De préférence, le propulseur est mis en mouvement par le relâchement d'une énergie potentielle, dans un mode de réalisation préféré, un ressort. Cette énergie peut être de forme variée, ressort plastique, ressort à lamelle, cartouche de gaz, air comprimé, force électromagnétique, génération de gaz par réaction chimique entre au moins deux composés.

Dans un mode de réalisation possible, la source d'énergie peut être l'opérateur lui-même. En appuyant avec une force suffisante, il générera par un mécanisme interne au dispositif la vitesse nécessaire à la pénétration de la ou des aiguilles dans le tissu. Le dispositif comprend également un mécanisme de sécurité, permettant de verrouiller le moyen d'activation afin d'éviter un déclenchement involontaire. Le mécanisme de sécurité peut se présenter sous différentes formes, l'appui de la bague ou du dispositif sur le tissu peut également déverrouiller la détente et permettre le déclenchement.

De préférence, le dispositif comprend aussi un récipient utilisé pour stocker la solution à administrer. Ce récipient peut être positionné sur le corps du dispositif ou être directement intégré au dispositif. L'activation de ce récipient, qui provoquera l'injection de la substance dans le tissu, peut être soit manuelle, soit automatique.

Dans ce deuxième cas, l'activation du dispositif entraînera, une fois les aiguilles en place, l'activation du récipient. Ce récipient peut prendre diverses formes telles que - et ceci de façon non exhaustive - un réservoir semi rigide, une poche souple, une seringue, une carpule.

Dans un mode de réalisation possible, avant l'injection et avant l'activation des moyens de propulsion, l'opérateur peut vider l'air éventuellement contenu dans la connexion fluidique ou le récipient. Le dispositif peut comprendre une fenêtre à hauteur de l'aiguille, permettant ainsi de voir la première goûte, preuve que la connexion fluidique est vide d'air.

Le dispositif peut comprendre un mécanisme qui rend difficilement accessible l'aiguille de manière à prévenir les blessures, avant et/ou après l'insertion ainsi qu'après l'injection. Cette protection peut s'obtenir notamment en rétractant l'aiguille dans le corps du dispositif, ou dans le support lui-même, ou en coulissant un cache qui empêche l'accès par inadvertance à l'aiguille. Un tel système de protection peut se déclencher automatiquement dès le retrait des micro-aiguilles du tissu du aptient, par exemple du fait de la force résiduelle.

Dans un mode de réalisation possible, l'extrémité distale du dispositif est composée d'une zone de contact en forme de bague ou de pieds stabilisateurs permettant de positionner le dispositif sur le tissu selon un angle désiré et n'influant pas sur les caractéristiques mécaniques du tissu

### Liste des figures

L'invention sera mieux comprise ci-après au moyen de quelques exemples illustrés. Il va de soi que l'invention ne se limite pas à ces modes de réalisation.
Figure 1 : Vue d'ensemble de l'inserteur.
Figures 2 : Vue éclatée de l'inserteur et vue détaillée de l'ensemble piston-propulseur.
Figure 3 : Vue de coupe de l'inserteur avec et sans ressort (4a) utilisé comme moyen de pression.
Figure 4 : Inserteur chargé et prêt à être actionné.
Figures 5 : Inserteur en position 1.
Figures 6 : Inserteur en position 2.
Figures 7 : Inserteur en position 3.
Figures 8 : Lancement de l'ensemble Piston-Propulseur.
Figure 9 : Début d'insertion de l'ensemble Piston-Propulseur.
Figure 10 : Ensemble Piston-Propulseur en position 1.
Figure 11 : Ensemble Piston-Propulseur en position 2.
Figure 12 : Ensemble Piston-Propulseur en position 3.

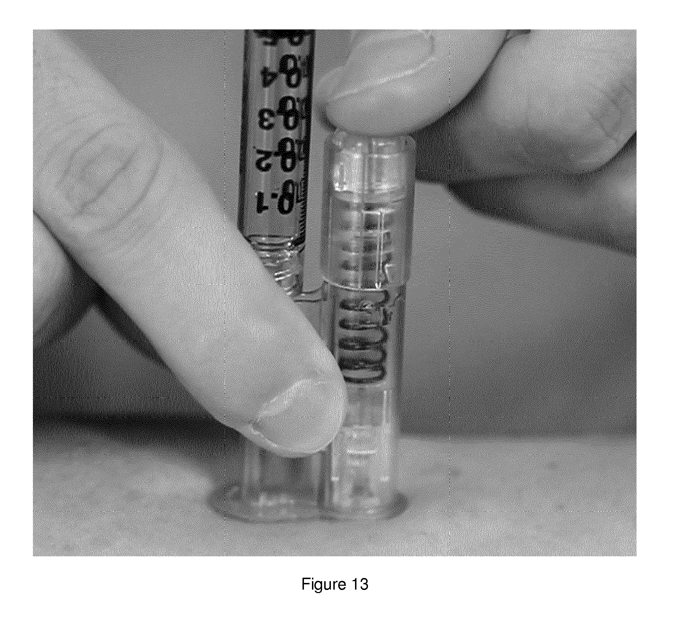

Figure 13 : Inserteur en après actionnement du propulseur.

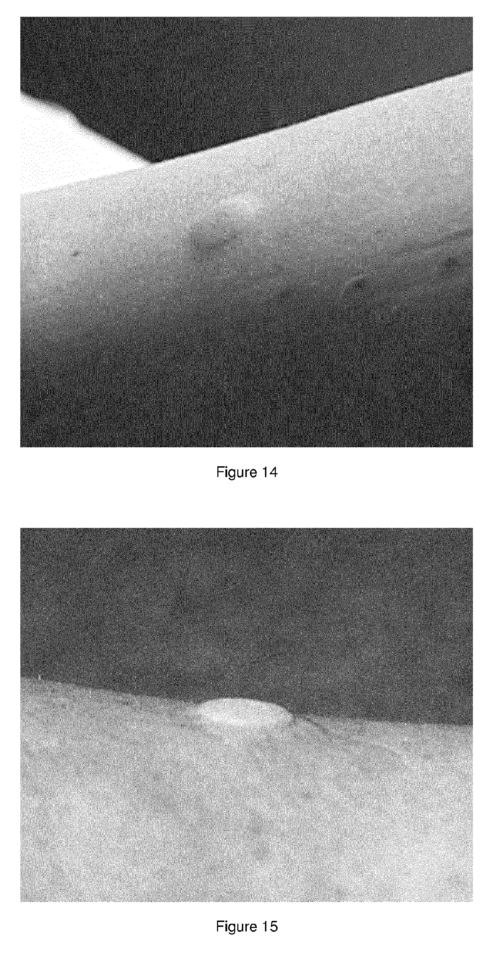

Figure 14 : Papule formée après injection de 0.2 ml de solution.
Figure 15 : Papule formée après injection de 0.5 ml de solution.

### Description détaillée de l'invention

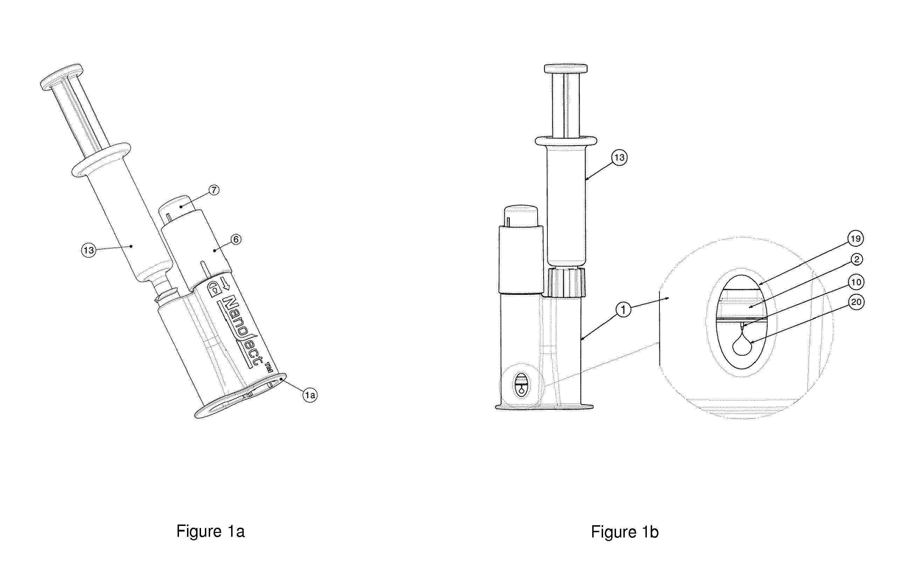

Les figures 1a et 1b représentent les différents composants du dispositif complet vu de l'extérieur avant l'insertion : le boitier (1), la face distale du dispositif (1a), le déclencheur (7) et l'élément de sécurité du déclencheur (6).

Afin de faire le vide d'air de la connexion fluidique entre la seringue (13) et l'aiguille (10) avant l'injection, le dispositif dispose d'une fenêtre (19) pour permettre de voir l'aiguille (10) et de voir la première goute (20) de la solution avant d'effectuer l'insertion.

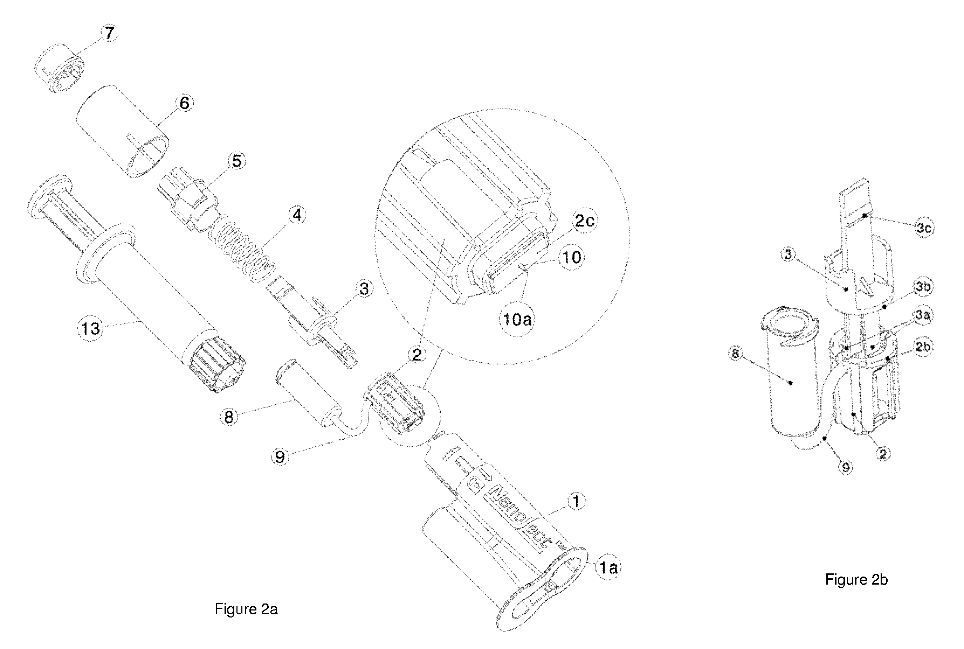

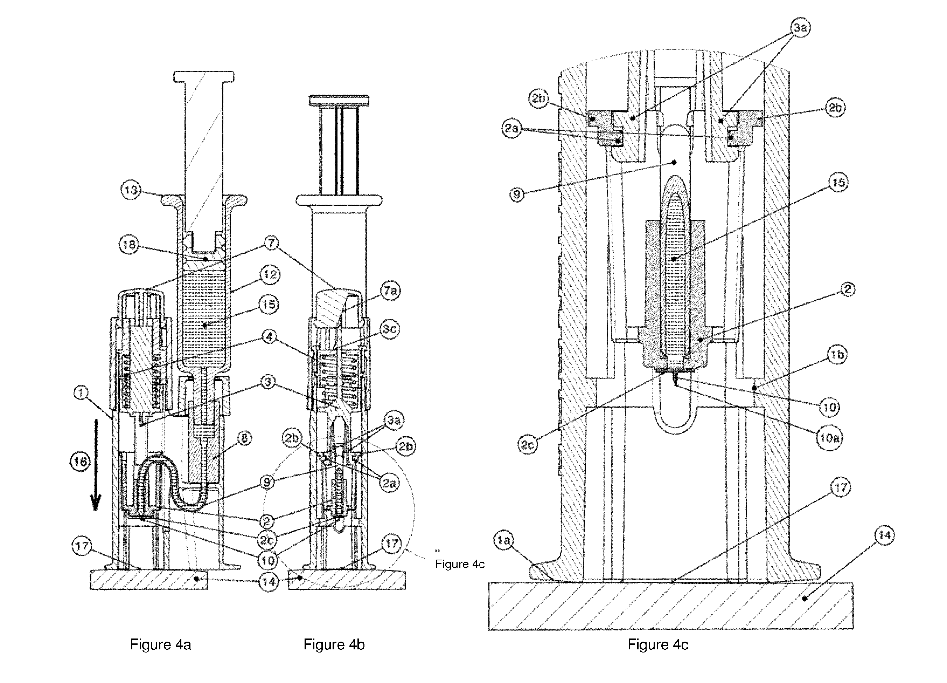

La figure 2a est une vue en éclaté du dispositif qui divulgue les différents éléments présents à l'intérieur :
- Le lueur (8), qui permet la connexion avec la seringue (13), est en connexion fluidique avec l'aguille (10) grâce à la tubulure (9).
- Les moyens de propulsion sont constitués d'un ressort (4), qui est la principale source d'énergie pour l'insertion, comprimé entre un élément solidaire au boitier (5) et le propulseur (3) monté mobile dans le boitier. Une telle disposition assure un mouvement guidé par la paroi intérieure du boîtier selon une direction correspondant à l'orientation principale (16 représenté sur la figure 4).
- L'aiguille (10) est solidaire du piston (2) et fait saillie par rapport à la face distale du piston (2c). L'aiguille (10) dispose elle-même d'une extrémité distale (10a) pointue. La taille de l'aiguille est fonction des caractéristiques du tissu où doit être effectuée l'injection. La distance entre la face distale (10a) de l'aiguille et celle (2c) du piston représente la profondeur à laquelle doit être effectué l'injection. La taille de la face distale (2c) permet de garantir cette profondeur maximum.

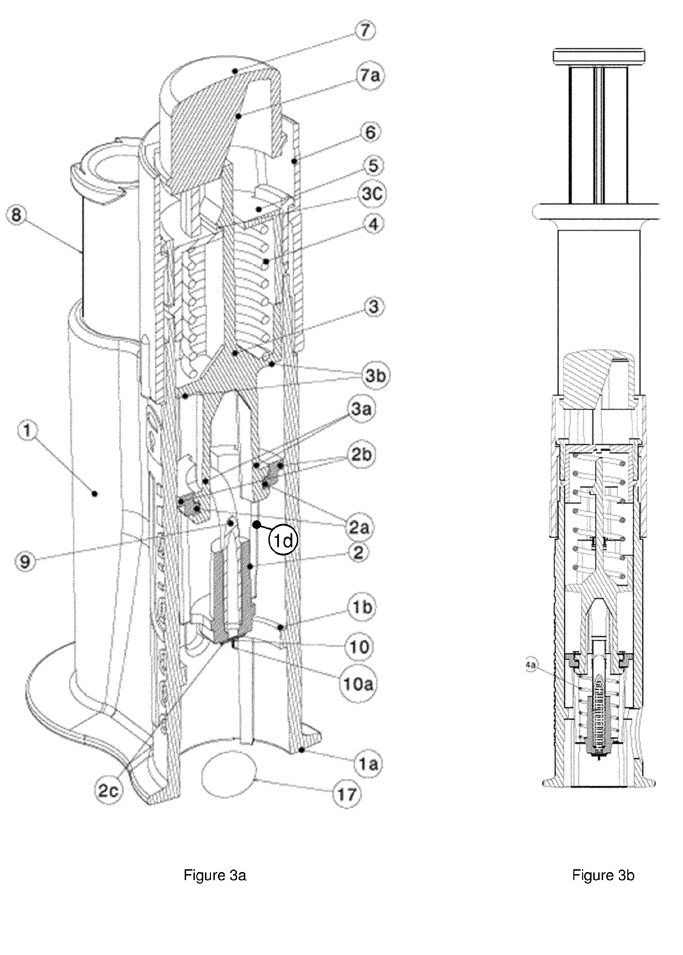

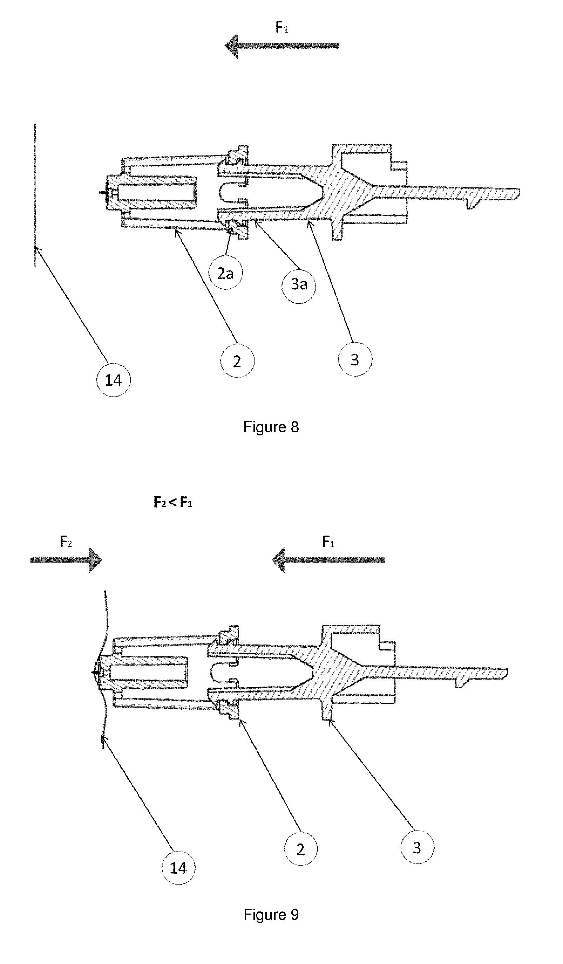

La figure 2b présente l'ensemble piston-propulseur (2, 3). Le propulseur dispose de moyens de retenue (3a) permettant de rendre temporairement solidaire cet ensemble piston-propulseur (2, 3). Le piston est façonné de manière à recevoir les moyens de retenue (3a) grâce aux éléments de réception (2a représenté sur la figure 3a). La figure 8 permet de voir avec plus de précision comment le piston est rendu solidaire au propulseur. Les moyens de retenue (3a) disposent de deux extrusions (par moyens de retenue) chanfreinés afin de clipser le piston (2) au propulseur (3) lors du montage de l'ensemble et de déclipser avant l'injection.

Les figures 3a et 3b représentent l'inserteur chargé, c'est-à-dire ayant le ressort (4) comprimé et prêt à effectuer l'insertion. La figure 3b se distingue de la figure 3a uniquement grâce à la présence du ressort (4a) qui agit comme moyens de pression.

Selon la figure 3a, l'aiguille (10) est solidairement fixée au piston (2) lui-même solidaire du propulseur (3). Le ressort (4) est comprimé entre le propulseur (3) et un élément solidaire du boitier (5). Cette position appelée « dispositif chargé » est maintenu temporairement par l'élément de retenue (3c) à l'élément solidaire du boitier (5). L'opérateur doit déverrouiller l'élément de sécurité (6) pour enfoncer le déclencheur (7) qui , à son tour, libère l'élément de retenue (3c).

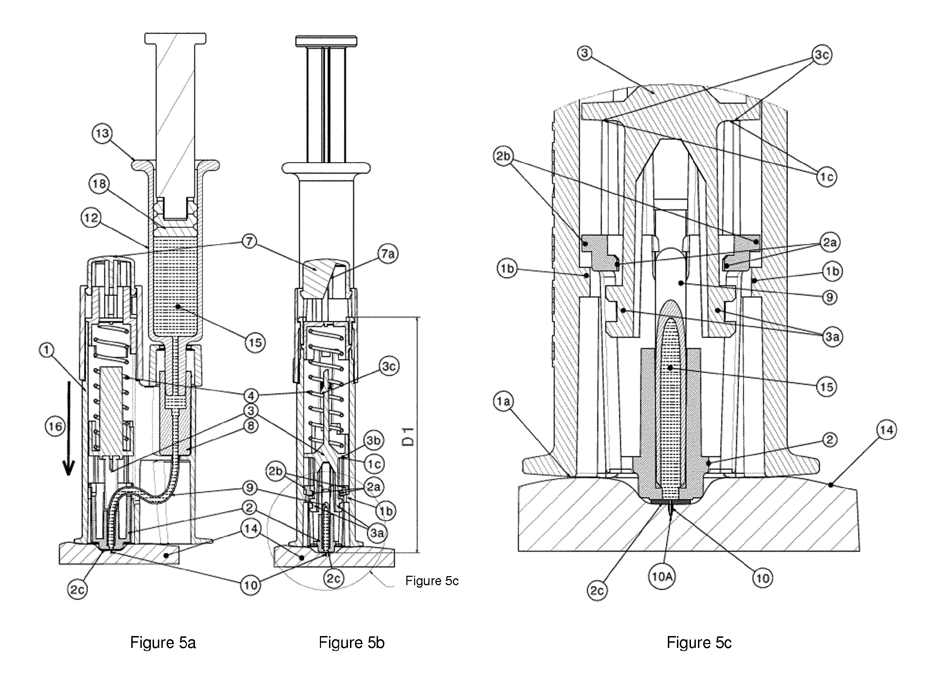

Le boitier dispose de moyens de guidage (1d) contraignant le piston à un mouvement selon la direction principale afin de venir en contact sur une zone prédéfinie (17). Le boitier contient également des butées (1b) et (1c représenté sur la figure 5) limitant respectivement la course du piston et du propulseur. La butée (1b) permet néanmoins à ce que la face distale (2c) du piston dépasse l'extrémité distale (1a) du dispositif et peut contraindre les éléments de retenue (3c) à se déclipser si la résistance du tissu n'a permis ce déclipsage avant. La butée (1c) maintient le propulseur (3) afin qu'il n'exerce plus de pression via son ressort (4) directement sur le piston (2).

Les figures 4 à 7 divulguent le dispositif complet du positionnement à l'injection.

Les figures 4a, 4b et 4c représentent le dispositif positionné sur le tissu afin d'injecter la solution sous la zone de contact (17). L'ensemble piston-propulseur (2, 3) est solidaire, le ressort (4) compressé. A ce stade, l'opérateur peut exercer une légère pression sur le piston (18) de la seringue afin de faire le vide d'air de la connexion fluidique comprenant le lueur (8) et la tubulure (9). Une fenêtre (19 représenté sur la figure 1b) permet de vérifier lorsque la première goute (20) de la solution (15) sort de l'aiguille (10).

Les figures 5a, 5b et 5c représentent le dispositif avec le piston (2) en position 1 formant ainsi une distance D1 entre l'extrémité proximale du boitier et la face distale (2a) du piston. Selon cette configuration, le piston a ainsi atteint sa position la plus éloigné de l'extrémité proximale du boitier. Il est toutefois possible que l'opérateur exerce une telle pression sur le dispositif qu'un bourrelet artificielle se forme. Dans ce cas, D1 pourrait ne pas être la distance maximale entre l'extrémité proximale du boitier et la face distale (2a) du piston.

A ce stade, les moyens de retenue (3a) peuvent se déclipser libérant ainsi le piston (2) de toute force exercée directement par le propulseur (3). Le déclipsage peut être causé par la résistance élastique du tissu ou par la butée 1b, tous deux exerçant une force s'opposant à la force exercée par le propulseur. Toutefois, l'ensemble aiguille-piston (10, 2) est contraint par des moyens de pression (par exemple 4a représenté sur la figure 3b) exerçant une force dans le sens de l'insertion (16) afin de maintenir l'aiguille (10) correctement insérée dans le tissu (14). A noter que la forme en S couché de la tubulure (9) peut également définir les moyens de pression. Les moyens de pression peuvent être actif dès l'activation du dispositif ou au moins dès que la face distale (10a) de l'aiguille touche le tissu (14).

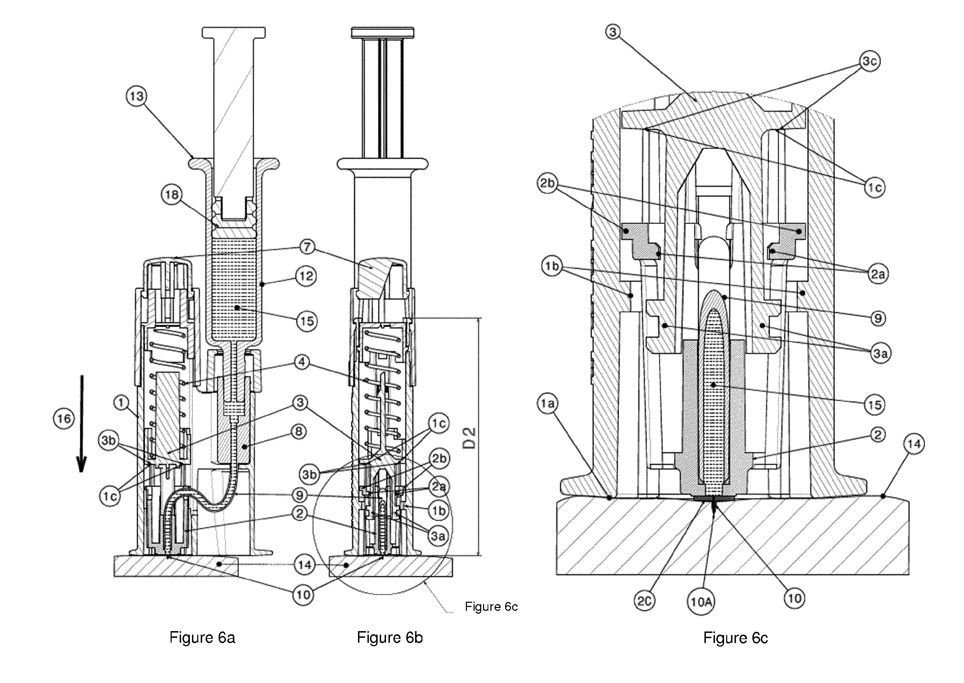

Les figures 6a, 6b et 6c représentent le dispositif avec le piston (2) en position 2 formant ainsi une distance D2 entre l'extrémité proximale du boitier et la face distale (2a) du piston. Cette position correspond normalement à un retour à l'équilibre où seuls les moyens de pression du dispositif exercent une force dans le sens de l'insertion (16).

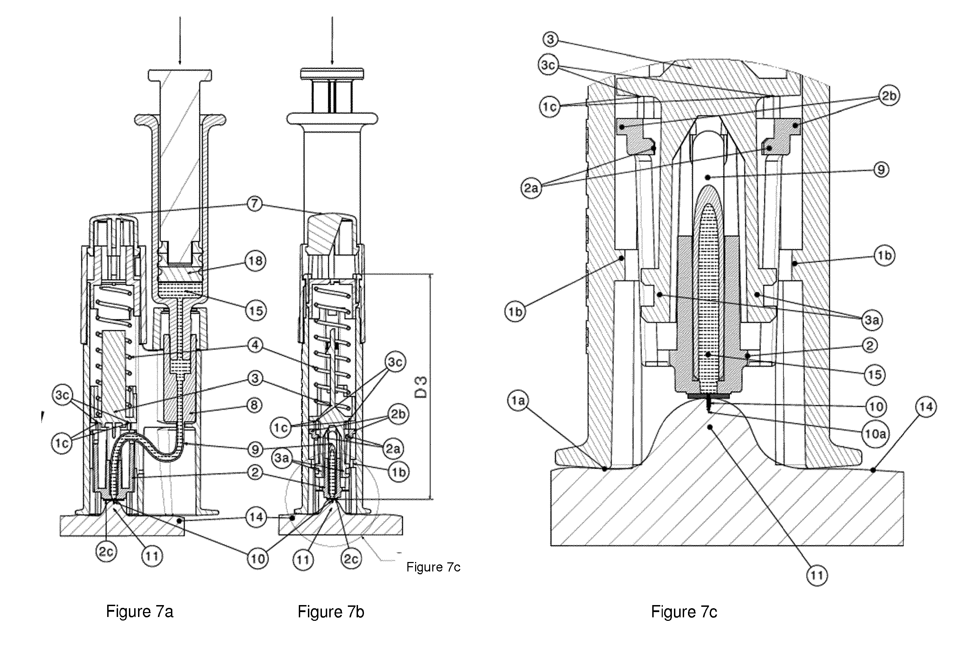

Les figures 7a, 7b et 7c représentent le dispositif avec le piston (2) en position 3 formant ainsi une distance D3 entre l'extrémité proximale du boitier et la face distale (2a) du piston. Cette distance D3 est inférieure à D1 et/ou D2. A ce stade, l'opérateur exerce une pression sur le piston (18) de la seringue (13), injectant la solution (15) sous la zone de contact (17). L'injection a pour conséquence une accumulation de la solution (15) sous la zone de contact (17) déformant cette dernière et formant ainsi une papule (11). Les moyens de pression sont configurés pour exercer une pression minimale afin de ne pas s'opposer à la formation de la papule (11). L'ensemble aiguille-piston effectue ainsi un recul qui est maîtrisée grâce aux moyens de pression.

Les figures 8 à 12 divulguent l'interaction entre le piston (2) et le propulseur (3) du positionnement à l'injection.

La figure 8 présente le lancement de l'ensemble piston-propulseur solidaire grâce aux moyens de retenue (3a). Le lancement du propulseur génère une force F1 exercée par les moyens de propulsion et entraîne le piston (2) en direction du tissu.

La figure 9 présente l'aiguille qui commence à pénétrer le tissu. L'ensemble piston-propulseur est encore solidaire et exerce une force F1 contre le tissu. Les caractéristiques mécaniques du tissu engendrent une force de sens opposé appelée F2. Plus le piston avance contre le tissu plus la force F2 augmente.

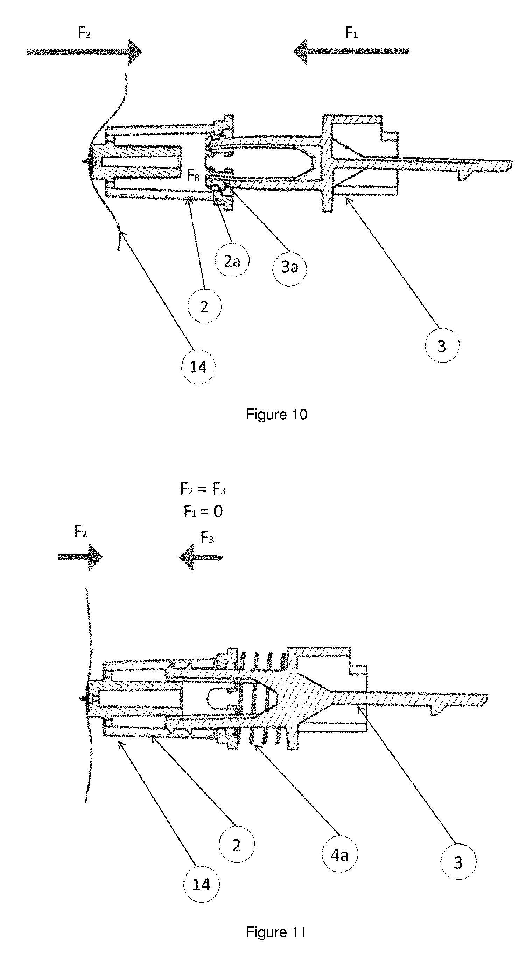

La figure 10 présente le piston en position 1. L'aiguille a parfaitement pénétré le tissu et la force F2 a atteint une valeur prédéterminée qui permet de déclipser les moyens de retenue. Exprimé différemment, les sens opposés des forces F2 et F1 induisent une force radiale Fr, dirigée selon une direction perpendiculaire par rapport à la direction des forces F1 et F2. Les forces F1, F2 et Fr font glisser le propulseur à l'intérieur du piston et rendre les moyens de retenue inopérants.

La figure 11 présente le piston en position 2 où F1 devient nulle car le piston est déconnecté du propulseur. Une nouvelle force appelée F3 est générée par les moyens de pressions (4a). F3 est égale à F2 ce qui correspond à un état d'équilibre. L'aiguille est insérée dans le tissu et les moyens de pressions (4a) maintiennent l'aiguille correctement insérée.

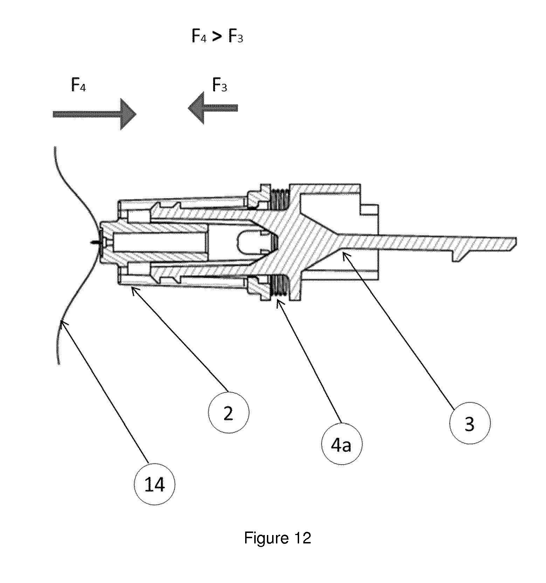

La figure 12 présente le piston en position 3. L'injection de la solution cause la formation d'une papule. Les moyens de pression exercent une force F3 inférieure à la force F4 générée par la formation de la papule. La force F3 permet de maintenir l'aiguille correctement insérée durant l'injection.

Il va de soi que l'invention ne se limite pas aux exemples illustrés et aux modes de réalisation divulgués dans le présent document.

### Références numériques utilisées dans les figures

1. Boitier
   1a Face distale du dispositif
   1b Elément de butée pour le piston
   1c Elément de butée pour le propulseur
   1d Moyens de guidage
2. Piston
   2a Eléments de réception des moyens de retenue du propulseur
   2b Eléments de retenue limitant la course du piston
   2c Face distale du piston
3. Propulseur
   3a Moyens de retenue du propulseur au piston
   3b Eléments de retenue limitant la course du propulseur
   3c Moyens de retenue du propulseur au boitier
4. Ressort du moyen de propulsion
   4a Ressort du moyen de pression
5. Elément solidaire du boitier
6. Elément de sécurité du déclencheur
7. Déclencheur
   7a Elément de déclenchement
8. Luer
9. Tubulure
10. Aiguille
   10a Extrémité distale de l'aiguille
11. Papule
12. Réservoir
13. Seringue
14. Tissu
15. Solution
16. Sens de l'insertion
17. Zone de contact
18. Piston de la seringue
19. Fenêtre
20. Goute

## Revendications

1. Dispositif pour l'insertion d'aiguilles comprenant
a. un boitier (1) défini par une extrémité proximale et une extrémité distale (1a),
b. un piston (2) monté mobile à l'intérieur du boitier (1),
c. au moins une aiguille creuse (10), comportant une extrémité distale (10a), rendue solidaire du piston (2),
d. des moyens de propulsion (3, 4) adaptés pour générer une force F1 sur le piston (2), en direction de l'extrémité distale (1a) du dispositif,
e. lesdits moyens de propulsion (3, 4) comprenant des moyens de retenue (3a) pour rendre temporairement solidaires les moyens de propulsion (3, 4) au piston (2) pendant que ce dernier est propulsé par les moyens de propulsion (3, 4),
f. lesdits moyens de retenue (3a) étant configurés de manière à libérer le piston (2) des moyens de propulsion (3,4) lorsqu'une force F2 de valeur déterminée et de sens opposé à F1 s'exerce sur le piston (2),
g. le piston (2) comprenant une face distale (2c) sur laquelle ladite aiguille (10) fait saillie,
h. le dispositif étant configuré de manière à ce que le piston (2) atteigne une première position, consécutivement à l'activation des moyens de propulsion (3, 4), formant une distance D1 entre la face distale du piston (2) et l'extrémité proximale du boitier (1) ; puis une deuxième position avant l'injection, formant une distance D2 entre la face distale du piston (2) et l'extrémité proximale du boitier (1) ; puis une troisième position, consécutivement à l'injection d'au moins une solution (15) dans ou à travers le tissu (14), formant une distance D3 entre la face distale du piston (2) et l'extrémité proximale du boitier (1) ; D3 étant inférieure ou égale à D1 et à D2 ;
i. le dispositif comportant en outre des moyens de pression destinés à maintenir ladite aiguille au moins partiellement insérée dans le tissu et à autoriser au moins un recul maîtrisé du piston (2) lors du passage de la première à la deuxième position puis à la troisième position.

2. Dispositif selon la revendication 1 agencé de manière à ce que D1 soit supérieure à D2.

3. Dispositif selon la revendication 1 agencé de manière à ce que D1 soit inférieure ou égale à D2.

4. Dispositif selon l'une quelconque des revendications précédentes dans lequel lesdits moyens de pression sont adaptés de manière à exercer une force dès l'activation des moyens de propulsion (3, 4).

5. Dispositif selon l'une quelconque des revendications précédentes dans lequel lesdits moyens de pression sont adaptés de manière à s'activer automatiquement dès que l'extrémité distale (10a) de l'aiguille est appuyée sur le tissu (14).

6. Dispositif selon l'une quelconque des revendications précédentes dans lequel les moyens de propulsion comprennent un propulseur (3) qui est fixé au moins temporairement par des moyens de retenue (3c) au boitier (1) ou à un élément (5) solidaire du boitier (1).

7. Dispositif selon l'une quelconque des revendications précédentes dans lequel la paroi intérieure du boitier et la paroi extérieure du piston sont configurées de manière à restreindre le piston à un mouvement dans l'axe de l'orientation principale (16).

8. Dispositif selon l'une quelconque des revendications précédentes dans lequel les moyens de propulsion (3, 4) sont composés d'au moins un propulseur (3) et d'un ressort (4), d'un élastique ou d'une lame élastique.

9. Dispositif selon l'une quelconque des revendications précédentes dans lequel les moyens de propulsion (3, 4) sont adaptés pour exercer une force d'une valeur supérieure ou égale aux moyens de pression.

10. Dispositif selon l'une quelconque des revendications précédentes dans lequel les moyens de pression exercent une force F3 de même sens que F1; F3 étant générée uniquement ou cumulativement par une tubulure (9), un ressort (4a), un élastique, une cartouche de gaz, de l'air comprimé, une force électromagnétique, la génération de gaz par réaction chimique entre au moins deux composés, une lame élastique, le poids de l'ensemble piston-aiguille (2, 10) et/ou induite par le frottement du piston contre les parois internes du boitier (1).

11. Dispositif selon la revendication 10 adapté de manière à ce que F3 soit inférieure à une force maximale F4 exercée contre la face distale du piston (2) qui est induite par la création de la papule consécutivement à l'injection de ladite solution (15).

12. Dispositif selon l'une quelconque des revendications précédentes dans lequel, l'extrémité distale (1a) est composée d'une zone de contact en forme de bague ou de pieds stabilisateurs permettant de positionner le dispositif sur le tissu (14) selon un angle désiré et n'influant pas sur les caractéristiques mécaniques du tissu (14).

13. Dispositif selon l'une quelconque des revendications précédentes comprenant une seringue (13) comportant un piston (18) destiné à expulser à travers l'aiguille (10) une solution (15) manuellement ou automatiquement dès que le piston (2) ait atteint ladite deuxième position.

14. Dispositif selon la revendication 13 dans lequel la seringue (13) est orientée selon une direction distincte de celle définie par le déplacement de l'aiguille (10).

15. Dispositif selon l'une quelconque des revendications précédentes comprenant une tubulure (9) assurant une communication fluidique entre un réservoir et l'aiguille (10).

16. Dispositif selon l'une quelconque des revendications précédentes comprenant une fenêtre (19) ménagée sur le boitier (1) à hauteur de l'aiguille lorsque cette dernière se trouve dans une position de repos, préalablement à l'activation des moyens de propulsion (3,4).

17. Dispositif selon l'une quelconque des revendications précédentes dans lequel le boitier (1) comporte un premier élément de butée (1b) disposé de manière à retenir le piston (2) lorsqu'il est entraîné vers ladite extrémité distale.

18. Dispositif selon la revendication 17 dans lequel le boitier comporte un deuxième élément de butée (1c) disposé de manière à retenir le propulseur (3) lorsqu'il est entraîné vers ladite extrémité distale (1a).

19. Utilisation d'un dispositif tel que défini dans l'une quelconque des revendications précédentes et les étapes suivantes :
a) propulsion d'au moins une aiguille (10),
b) déplacement de l'aiguille induit par une première force externe au dispositif,
c) injection d'au moins une solution à travers l'aiguille (10).

20. Utilisation selon la revendication 19 dans laquelle les moyens de retenue libère le piston lors de l'étape b) ou durant la transition de l'étape a) à l'étape b) ou de l'étape b) à l'étape c),

21. Utilisation selon la revendication 19 ou 20 dans laquelle ledit déplacement de l'étape b) est maîtrisé grâce aux moyens de pression.

22. Utilisation selon l'une quelconque des revendications 19 à 21 dans laquelle lesdites étapes sont réalisées successivement.

23. Utilisation selon l'une quelconque des revendications 19 à 21 dans laquelle les étapes b) et c) sont réalisées simultanément.

24. Utilisation selon l'une quelconque des revendications 19 à 24 comprenant en outre l'étape suivante :
d) recul de l'aiguille induit par une deuxième force externe au dispositif.

25. Utilisation selon la revendication 24 dans laquelle ledit recul, de l'étape d), est maîtrisé grâce aux moyens de pression.
